# EUROPEAN PATENT APPLICATION

(11) **EP 4 596 708 A1**
(43) Date of publication of application: **06.08.2025**
(21) Application number: 22960269.3
(22) Date of filing: 30.09.2022
(51) Int. Cl.: C12Q 1/68, G01N 33/50

(54) **BIOCHIP AUTOMATIC CONTROL METHOD AND AUTOMATIC CONTROL SYSTEM THEREFOR**

(71) Applicant: BGI Shenzhen, Shenzhen, Guangdong 518083 (CN)
(72) Inventor: HE, Futao, Shenzhen, Guangdong 518083 (CN); LEI, Xiaojuan, Shenzhen, Guangdong 518083 (CN); CHEN, An, Shenzhen, Guangdong 518083 (CN); QIU, Zhifeng, Shenzhen, Guangdong 518083 (CN); HONG, Yan, Shenzhen, Guangdong 518083 (CN); WAN, Zhiyuan, Shenzhen, Guangdong 518083 (CN); SHEN, Mengzhe, Shenzhen, Guangdong 518083 (CN); CHEN, Ao, Shenzhen, Guangdong 518083 (CN); LI, Yuxiang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Lavoix
(86) International application number: PCT/CN2022/123191
(87) International publication number: WO 2024/065646

(57) **Abstract**

The present disclosure relates to a method for automatically controlling a biochip and an automatic control system, wherein the method comprises the operations of carrying a suction member through a pipette mechanism to suck a reagent, and carrying the suction member that has sucked the reagent through the pipette mechanism to add the reagent to the biochip, and also provides an automatic control system of a biochip using the method for automatically controlling a biochip. The method and system of a biochip may automatically schedule and control a plurality of biochips, so as to complete the labeling and capturing of biological information in tissues, and perform overall collection of the captured products, which meet the requirements of high utilization rate and high degree of automation for a biochip.

## Description

### TECHNICAL FIELD

The present disclosure relates to the field of automatic operation of a biochip, in particular to a method for automatically controlling a biochip and an automatic control system thereof.

### BACKGROUND

Spatial transcriptomics is to measure a total mRNA of a complete tissue section, combine the spatial information of the total mRNA with the morphological content, and draw the positions where all gene expressions occur, so as to obtain a complex and complete gene expression map of a biological process. Microscopy and sequencing technology may be combined to obtain gene expression data while preserving the spatial location information of samples to a maximum extent, which provides important information for the relationship between cell function, phenotype and location in tissue microenvironment.

Spatial transcriptomics technology obtains a spatial position and the corresponding gene expression information, and performs analysis on the spatial transcriptomics data so that it may be known which signal transduction pathways activate some cells. Scientists may select genes of interest through the data generated by spatial transcriptomics technology, and display their spatially resolved expressions on an original tissue section. Since all mRNAs are captured, it is possible to select any number of genes in the form of any combination so as to view and analyze together, but no longer limited to viewing only a single gene. These research processes are all required to transform the information in biological tissues into detectable forms through biochemical reactions, which involve multiple reagents and usually require cumbersome manual operations therein to repeat multiple rounds or multiple steps of biochemical reactions with different principles.

The inventors have found that, space research technology is mainly based on microfluidic chip, laser rodissection, targeted multiple staining, capture biochip with oligonucleotide chain modification and the like. 10X Genomics mainly provides a manual operation based solution, and supporting instrument solutions are provided: BondRX immunohistochemical staining instrument based on Leica of Nano String Company and laser capture dissection for multiple staining and imaging performed on GeoMx platform; multiple staining and in-situ imaging based on PhenoCycler-Fusion of Akoya Company. The above, which are all based on semi-automatic biochemical operation, microscopy and sequencing, based on full-automatic microscope and manual biochemical operation, or based on full-automatic multiple staining and microscopy of a single biochip, are difficult to incorporate the aspects of high degree of automation of biochemical reaction, high spatial resolution, large sample processing flux and wide capture range.

Further, the inventors have found that, the supporting instruments of spatial omics mainly focus on multiple staining, or post-staining imaging, or a combination thereof. These instruments which may only handle standard biochips with a length-width of 25mm*75mm, are not compatible with the application of biochips with other sizes. For other planar biochips, it cannot be adapted as well. At the same time, only a target region of interest may be selected to collect a target object so that it is difficult to collect all the products on a large scale. For example, it is not possible to perform the function of overall product collection for a flat biochip with a centimeter level length-width.

In addition, the inventors have found that, the solution containing collecting samples to perform mixed sequencing which needs manual time-consumption to select a target region of interest (ROI), may only be performed at a low flux, rather than performing high-flux processing of a biochip. The instruments found by the inventors generally require that the target biochip or the biochip needs an additional region for sealing and/or clamping, which cannot incorporate a high utilization rate and a minimized reagent consumption of a biochip at the same time. Halfway intervention by personnel is required.

### SUMMARY

In order to achieve the above-described object, the present disclosure provides a method for automatically controlling a biochip, comprising an operation, the operation comprising: carrying a suction member by means of a pipette mechanism to suck a reagent, and carrying the suction member that has sucked the reagent by means of the pipette mechanism to add the reagent to the biochip.

In some embodiments, the operation includes at least one of following steps:
incubation: the pipette mechanism carries the suction member to suck an incubation reagent, and the pipette mechanism carries the suction member that has sucked the incubation reagent to add the incubation reagent to the biochip;
tissue permeabilization: the pipette mechanism carries the suction member to suck a permeabilization reagent, and the pipette mechanism carries the suction member that has sucked the permeabilization reagent to add the permeabilization reagent to the biochip;
reverse transcription: the pipette mechanism carries the suction member to suck a reverse transcription reagent, and the pipette mechanism carries the suction member that has sucked the reverse transcription reagent to add the reverse transcription reagent to the biochip; and
tissue removal: the pipette mechanism carries the suction member to suck a tissue removal reagent, and the pipette mechanism carries the suction member that has sucked the tissue removal reagent to add the tissue removal reagent to the biochip.

In some embodiments, in each step of the operations, waste liquid is pumped by a waste liquid pumping mechanism.

In some embodiments, after the tissue removal step, it further comprises at least one of following steps:
nucleic acid release: the pipette mechanism carries the suction member to suck a nucleic acid release reagent, and the pipette mechanism carries the suction member that has sucked the nucleic acid release reagent to add the nucleic acid release reagent to the biochip so as to complete the nucleic acid release; and
product collection: the pipette mechanism carries the suction member to suck the released nucleic acid and store the released nucleic acid.

In some embodiments, a cleaning step is performed between the incubation step and the tissue permeabilization step and/or a cleaning step is performed between the tissue permeabilization step and the reverse transcription step and/or a cleaning step is performed between the reverse transcription step and the tissue removal step and/or a cleaning step is performed between the tissue removal step and the nucleic acid release step.

In some embodiments, the cleaning step is that: the pipette mechanism carries the suction member to suck a cleaning reagent, and the pipette mechanism carries the suction member that has sucked the cleaning reagent to add the cleaning reagent to the biochip, and the waste liquid pumping mechanism pumps the cleaning waste liquid.

In some embodiments, each step of the operations is automatically performed.

In some embodiments, in each step of the operations, a current step and/or a current state are displayed to the user in real time.

In some embodiments, before the operations, a plurality of biochips is arranged in a biochip kit, so that the information of the plurality of biochips is associated with the information of the biochip kit by scanning, and the permeabilization time of the plurality of biochips is input into the system.

In some embodiments, the biochip on which a target tissue is carried is provided with a customized size.

In some embodiments, the plurality of biochips is sequentially arranged in at least one row in the biochip kit.

In some embodiments, if the plurality of biochips has the same permeabilization time:
when a plurality of biochips is arranged in one row, simultaneously pumping waste liquid of the permeabilization reagent, simultaneously adding the cleaning reagent, simultaneously pumping waste liquid of the cleaning agent and simultaneously adding the reverse transcription reagent for the one row of biochips; and
when the plurality of biochips are arranged in two rows, pumping waste liquid of the permeabilization reagent and adding the cleaning reagent for the first row of biochips before pumping waste liquid of the permeabilization reagent and adding the cleaning reagent for the second row of biochips; then pumping waste liquid of the cleaning reagent and adding the reverse transcription reagent for the first row of biochips before pumping waste liquid of the cleaning reagent and adding the reverse transcription reagent for the second row of biochips.

In some embodiments, if the plurality of biochips does not have the same permeabilization time, and the plurality of biochips are arranged sequentially in an ascending order according to the permeabilization time,
when the plurality of biochips are arranged in one row, pumping waste liquid of the permeabilization reagent, adding the cleaning reagent, pumping waste liquid of the cleaning reagent, and adding the reverse transcription reagent sequentially for the plurality of biochips according to a temporal sequence of completion of the permeabilization time;
when the plurality of biochips are arranged in two rows, and the two rows of biochips do not have the same permeabilization time therebetween, pumping waste liquid of the permeabilization reagent, adding the cleaning reagent, pumping waste liquid of the cleaning agent and adding the reverse transcription reagent sequentially for each biochip among the first row of biochips according to a temporal sequence of completion of the permeabilization time before pumping waste liquid of the permeabilization reagent, adding the cleaning reagent, pumping waste liquid of the cleaning agent and adding the reverse transcription reagent sequentially for each biochip among the second row of biochips according to a temporal sequence of completion of the permeabilization time; and
when the plurality of biochips are arranged in two rows, if the two rows of biochips have the same permeabilization time therebetween, first pumping waste liquid of the permeabilization reagent, adding the cleaning reagent, pumping waste liquid of the cleaning agent and adding the reverse transcription reagent sequentially for other biochips than the biochips having the same time among the first row of biochips according to a temporal sequence of completion of the permeabilization time; after pumping waste liquid of the permeabilization reagent and adding the cleaning reagent for the biochips having the same time among the first row of biochips, pumping waste liquid of the permeabilization reagent and adding the cleaning reagent for the biochips having the same time among the second row of biochips; after pumping waste liquid of the cleaning agent and adding the reverse transcription reagent for the biochips having the same time among the first row of biochips, pumping waste liquid of the cleaning agent and adding the reverse transcription reagent for the biochips having the same time among the second row of biochips; then pumping waste liquid of the permeabilization reagent, adding the cleaning reagent, pumping waste liquid of the cleaning agent and adding the reverse transcription reagent sequentially for other biochips among the second row of biochips according to a temporal sequence of completion of the permeabilization time.

In some embodiments, the method for automatically controlling a biochip comprises a waste liquid pumping step, the waste liquid pumping step including:

In the case that the plurality of biochips are arranged in one row, moving the waste liquid pumping mechanism above the biochip kit, starting a pump of the waste liquid pumping mechanism to first pump the waste liquid at a higher position in the biochip kit and then pump the waste liquid at a lower position in the biochip kit through a waste liquid pumping member, raising the waste liquid pumping member along a Z axis and closing the pump, and moving the waste liquid pumping mechanism to a cleaning region for cleaning; and

In the case that the plurality of biochips are arranged in two rows, pumping the waste liquid reacted by the first row of biochips before pumping the waste liquid reacted by the second row of biochips, wherein the pumping step is the same as the pumping step when the plurality of biochips are arranged in one row.

In some embodiments, after inputting the permeabilization time of each biochip, the system scans the information of the biochip kit and the information of the biochip in the biochip kit to determine whether the position of the biochip in the biochip kit, the number of the biochip and the information of the biochip are matched with the information in the system;
if not matched, remind the user;
if matched, determine the states of the existing biochip kits in the system:
if some of the existing biochip kit are not in the reverse transcription step or the nucleic acid release step, remind the user that it is still necessary to wait for a duration of X before transferring the biochip kit to the reaction region to perform the operations; and
if all the existing biochip kits are in the reverse transcription step or the nucleic acid release step, determine whether there is a vacancy in the reaction region; if there is no vacancy, remind the user that it is still necessary to wait for a duration of Y before transferring the biochip kit to the reaction region; and if there is a vacancy, transfer the biochip kit to the reaction region to perform the operations.

In some embodiments, between the step of placing the biochip in the biochip kit and the operations, the biochip kit is transferred to the reaction region by a gripper mechanism to perform the operations, and the gripper mechanism and the pipette mechanism work independently of each other.

In some embodiments, in the step of the operations, neither the gripper mechanism nor the pipette mechanism touches the biochip.

In some embodiments, covering a sealing cover and/or a temperature control cover and opening a sealing cover and/or a temperature control cover are performed on the biochip kit by the gripper mechanism.

In some embodiments, in the product collection step, the biochip kit is first transferred to a slanted region by the gripper mechanism, and product collection is completed in the slanted region.

In some embodiments, in the product collection step, the pipette mechanism carries a suction member to suck a reagent, the pipette mechanism carries the suction member that has sucked the reagent to flush the biochip in the biochip kit, and the pipette mechanism carries the suction member to perform product collection.

In some embodiments, there comprises a step of retrieving a suction member by the pipette mechanism before the operations, the step including: determining whether a current column in a suction member container satisfies the conditions for retrieving a suction member, so that when the number and position satisfy the conditions, the pipette mechanism moves above the row of suction members and the heights of the suction members decrease, and at the same time the row of suction members are retrieved; if the current column does not satisfy the conditions for retrieving a suction member, a next column is polled until polling of the suction member container is completed, and polling of the next suction member container begins until polling of all the suction member containers is completed; and if there is still not a column that satisfies the conditions for retrieving a suction member, the user is reminded to replenish a suction member.

In some embodiments, the step of sucking a reagent by the pipette mechanism includes that: the pipette mechanism carries the suction member to transfer to above the reagent region and the height of the suction member decreases, a reagent level is detected, so that if a reagent is not detected, remind the user to replenish a reagent; if a reagent is detected, determine whether a surplus amount satisfies an amount to be sucked by the carried suction member; if not, remind the user to replenish a reagent; if yes, further determine whether the reagent to be added is a cleaning reagent; if it is a cleaning reagent, the heights of all the suction members decrease to suck the cleaning reagent together; if it is not a cleaning reagent, the heights of all the suction members decrease respectively to suck the reaction reagent.

In some embodiments, the method is applied to labeling or capturing of transcriptomics, proteomics, metabonomics and/or lipidomics as well as pathological labeling.

The present disclosure also provides an automatic control system of a biochip, wherein the automatic control system comprises a biochip kit, a reaction region, a pipette mechanism and a gripper mechanism, and is configured to perform the above-described method for automatically controlling a biochip.

The method and the automatic control system provided by the present disclosure may realize automatic scheduling and control extensible for a plurality of biochips. The labeling and capture of the interstitial biological information, as well as the analysis of multi-omics such as transcriptome, proteome and immunohistochemistry is analyzed, and the collection function of the captured products is completed.

The method and the system provided by the present disclosure satisfy the processing requirements of high utilization rate, high flux and high degree of automation degree for a plurality of biochips.

### BRIEF DESCRIPTION OF THE DRAWINGS

Fig. 1 is an overall flowchart of a method for automatically controlling a biochip according to the present disclosure;
Fig. 2 is a flowchart of a pipette tip of a method for automatically controlling a biochip according to the present disclosure;
Fig. 3 is a flowchart of adding a reagent in a method for automatically controlling a biochip according to the present disclosure;
Fig. 4 is a flowchart of an incubation step of a method for automatically controlling a biochip according to the present disclosure;
Fig. 5 is a flowchart of a cleaning step of a method for automatically controlling a biochip according to the present disclosure;
Fig. 6 is a flowchart of a tissue permeabilization step of a method for automatically controlling a biochip according to the present disclosure;
Fig. 7 is a schematic view of two rows of biochips with different permeabilization time;
Fig. 8 is a flowchart of s reverse transcription step of a method for automatically controlling a biochip according to the present disclosure;
Fig. 9 is a flowchart of a tissue removal step of a method for automatically controlling a biochip according to the present disclosure;
Fig. 10 is a flowchart of a nucleic acid release step of a method for automatically controlling a biochip according to the present disclosure;
Fig. 11 is a flowchart of a collection step of a method for automatically controlling a biochip according to the present disclosure; and
Fig. 12 is a flowchart of a waste liquid pumping step of a method for automatically controlling a biochip according to the present disclosure.

### DETAILED DESCRIPTION

In the following, the present disclosure will be further described in detail in conjunction with specific embodiments, and the examples are provided only to elaborate the present disclosure, rather than limiting the scope of the present disclosure.

The present disclosure provides a method for automatically controlling a biochip and an automatic control system for implementing the method.

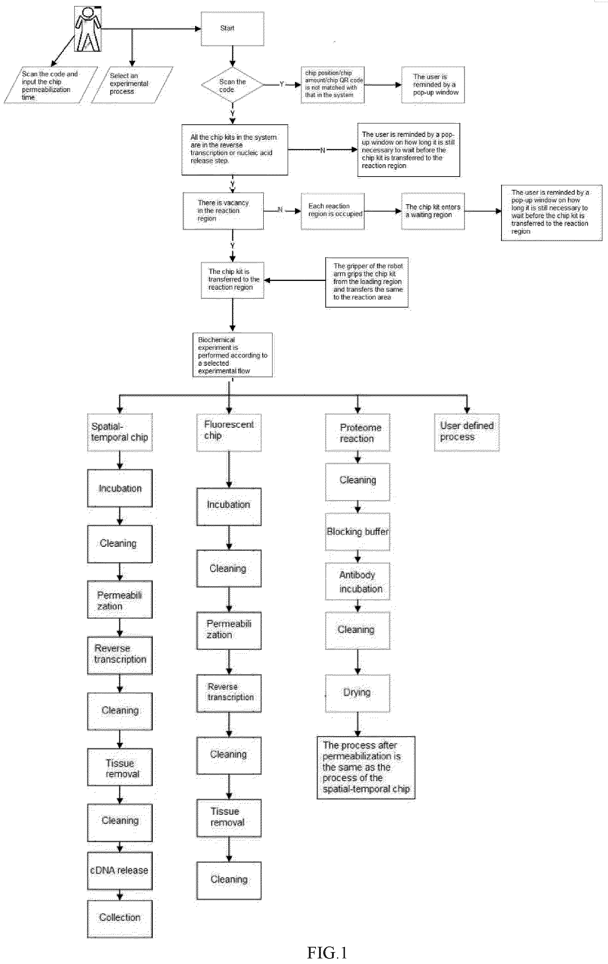

As shown in Fig. 1, the method for automatically controlling a biochip the present disclosure includes a prior-to-performing stage and a performing stage.

In the prior-to-performing stage, the following steps may be operated:

Outside the system, a single biochip is bound to a biochip kit by scanning a code, and the permeabilization time of each biochip is input, for example, by manual input.

A performing consumable (a suction member such as a pipette tip and a reagent) is placed, for example, by manual placement.

The performing process of the software interface is selected, and the start button is clicked to enter an automatic running process:

Starting from scanning a code, scan a QR code outside the biochip kit, and scan a biochip QR code in the biochip kit to determine whether the biochip position/biochip number/biochip QR code is matched with the information in the system (manually entered/obtained from a docking system). If not matched, the user is reminded by a pop-up window of the software that: the biochip information is not matched with the information in the system, and the process ends; if matched, enter a next process.

When scanning a code is completed and the biochip information is matched with the system, the program enters a process of determining a state of the biochip kit in the system. If some of the biochip kits in the system are not in the reverse transcription or nucleic acid release stage, the user is reminded by a pop-up window that: it is still necessary to wait for a duration of X before transferring the biochip kit to the reaction region. If all the biochip kits in the system are in the reverse transcription or nucleic acid release stage, enter a next judgment flow.

It is determined whether there is a vacancy in the reaction region. If all the reaction regions are occupied, the biochip kit will enter a waiting region, and the user is reminded by a pop-up window that: it is still necessary to wait for a duration of Y before transferring the biochip kit to the reaction region. If there is a vacancy in the reaction region, the program enters a next flow.

A gripper mechanism is controlled to grip the biochip kit from the loading region and transfer the same to the reaction region, and the gripper mechanism and a pipette mechanism work independently. Alternatively, the gripper mechanism includes a gripper, and the pipette mechanism includes a multi-channel pipette robot arm.

The system performs biochemical reaction according to a selected performing flow.

In the method according to the present disclosure, it is only necessary to manually input the performing information and place a performing consumable and a reagent in the prior-to-performing stage. Manual intervention is not required in the whole performing stage. The software interface may display a currently performed step and state in real time and may display estimated remaining performing time. The operator may predict and arrange operations in advance as it depends, so as to save the manpower.

The capture and product collection of spatial omics (for example, transcriptomics, proteomics, metabonomics and lipidomics) are realized in the performing stage, which includes the following steps:

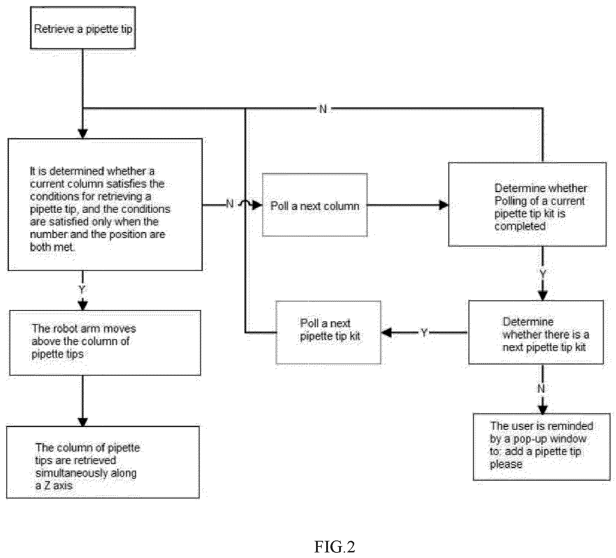

In some embodiments, as shown in Fig. 2, the step of retrieving a pipette tip includes: determining whether a current column satisfies the conditions for retrieving a pipette tip, and the conditions are satisfied only when the number and the position are both met. The pipette mechanism moves above the column of pipette tips, and drop along the Z axis direction (height direction), and a pipette tip is retrieved in the column at the same time. If the current column does not satisfy the conditions for retrieving a pipette tip, a next column is polled until polling of the pipette tip kit is completed, and polling of a next pipette tip kit begins until polling of all the pipette tip kits is completed; and if there is still not a column that satisfies the conditions for retrieving a pipette tip, the user is reminded by a pop-up window to: replenish a pipette tip please.

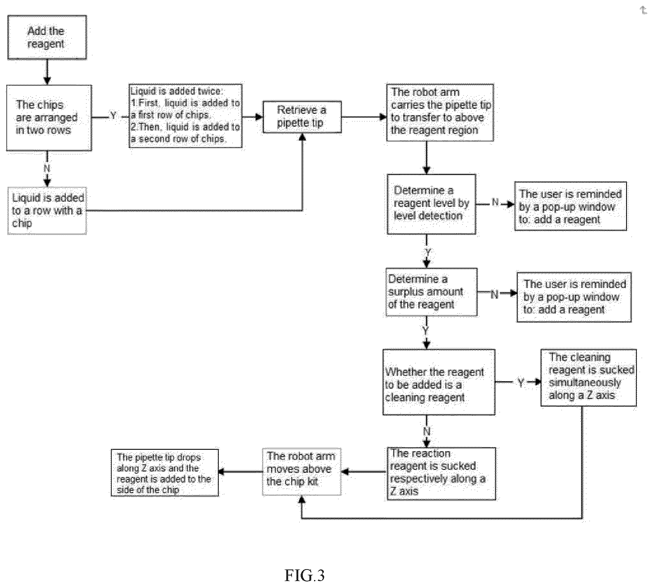

In some embodiments, as shown in Fig. 3, the step of adding a reagent includes: determining whether the biochips in the biochip kit are one row or two rows:

If the biochips are arranged in only one row, it suffices to add a liquid to the one row of biochips once.

If the biochips are arranged in two rows, first add a liquid to a first row of biochips: a pipette tip is retrieved for the first row of biochips, the pipette mechanism carries the pipette tip to transfer to above the reagent region and drop along the Z axis direction (height direction), and a reagent level is detected. If a reagent is not detected, the user is reminded by a pop-up window to: replenish a reagent please. If it is detected, it is determined whether the surplus amount is sufficient for the amount to be sucked by the carried pipette tip. If not sufficient, the user is reminded by a pop-up window to: replenish a reagent please. If the surplus amount is sufficient, it is determined whether the reagent to be added is a cleaning reagent. If it is a cleaning reagent, all pipette tips drop along a Z axis direction (height direction) to suck the cleaning reagent together, and the cleaning reagent is loaded into a large-capacity container. If it is not a cleaning reagent, the pipette tips drop along a Z axis direction (height direction) respectively, and the reaction reagent is sucked, and the reaction reagent is located in a small-capacity orifice plate. After a reagent is sucked, the pipette mechanism carries the pipette tip to transfer to above the biochip kit of the reaction region, and drops along a Z axis direction (height direction), and the reagent is added to the side of the biochip. Next, a liquid is added to the second row of biochips, which is the same as the step of adding a liquid for the first row of biochips.

In some embodiments, in the step of adding a reagent, if the biochips are arranged in three or more rows, the above-described step of adding a reagent for the biochips which are arranged in only one row is performed sequentially on three or more rows of biochips.

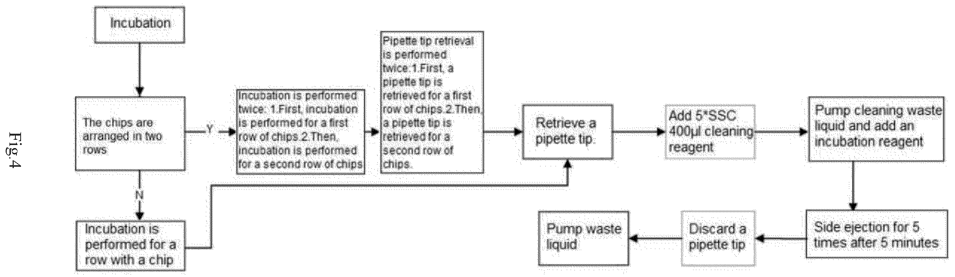

In some embodiments, as shown in Fig. 4, the incubation step includes: determining whether a plurality of biochips are arranged in one row or two rows in the biochip kit:

If the biochips are arranged in only one row, a pipette tip is retrieved for this row of biochips (the process of retrieving a pipette tip is shown in Fig. 3), the pipette mechanism such as a multi-channel pipette robot arm carries a pipette tip to the reagent region, sucks a certain amount of cleaning reagent with a high concentration (for example, 5*SSC 400µL) (see Fig. 3 for a process of sucking a reagent), and add the cleaning reagent to this row of biochips. Then, the waste liquid pumping mechanism pumps waste liquid of the cleaning reagent. Next, the pipette mechanism carries a plurality of pipette tips above the reaction region, drops along a Z axis direction (height direction), sprays an incubation reagent to the side of the biochip, and performs side ejection for multiple times, for example, 5 times after a predetermined duration, for example, 5 minutes. The pipette mechanism carries a plurality of pipette tips above a dust bin, and discards the pipette tips. After the incubation reagent is added, the suction member of the waste liquid pumping mechanism, for example, a needle, proceeds above the biochip kit, and the needle descends to pump waste liquid of the incubation reagent for this row of biochips.

If a plurality of biochips are arranged in two rows, a pipette tip is retrieved first for a first row of biochips (the process of retrieving a pipette tip is seen in Fig. 3), the pipette mechanism such as a multi-channel pipette robot arm carries a pipette tip to the reagent region, sucks a certain amount of cleaning reagent with a high concentration (for example, 5*SSC 400µL) (see Fig. 3 for a process of sucking a reagent), and add the cleaning reagent to this row of biochips. Then, the waste liquid pumping mechanism pumps waste liquid of the cleaning reagent. Next, the pipette mechanism carries the pipette tips above the reaction region, descends along a Z axis height direction, releases an incubation reagent to the side of the biochip, and performs side ejection for multiple times, for example, 5 times after a predetermined duration, for example, 5 minutes. The pipette mechanism carries a plurality of pipette tips above a dust bin, and discards the pipette tips. Next, a pipette tip is retrieved for a second row of biochips and the above-described steps are repeated. After the incubation reagent is added, the waste liquid pumping member of the waste liquid pumping mechanism, for example, a needle, proceeds above the biochip kit, and the needle descends to pump waste liquid of the incubation reagent for the same row of biochips at the same time.

In some embodiments, in the incubation step, if it is determined that the biochips are arranged in three or more rows, the incubation step for the above-described plurality of biochips which are arranged in one row is performed sequentially on three or more rows of biochips.

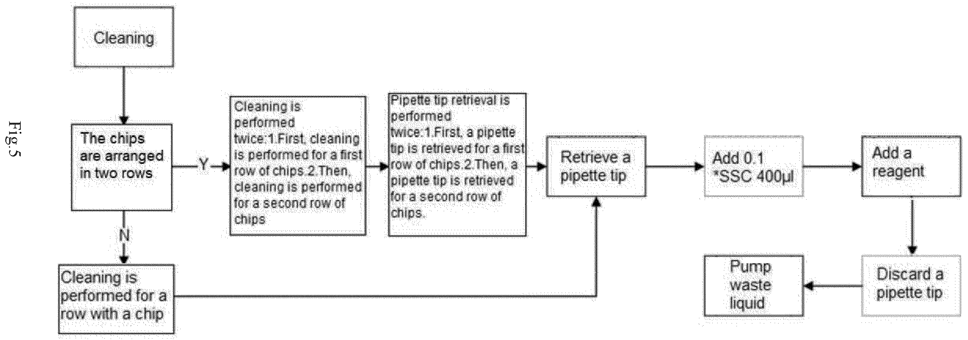

In some embodiments, as shown in Fig. 5, the cleaning step includes: determining whether the biochips are arranged in one row or two rows:

If the biochips are arranged in only one row, clean this row of biochips, retrieve a pipette tip, add a certain amount of cleaning reagent with a low concentration (for example, 0.1*SSC 400µL), discard the pipette tip, and pump waste liquid of the cleaning reagent.

If the biochips are arranged in two rows, first clean a first row of biochips, retrieve a pipette tip, add a certain amount of cleaning reagent with a low concentration (for example, 0.1*SSC 400µL), discard the pipette tip, and pump waste liquid of the cleaning reagent. Then, a second row of biochips are cleaned, similar to the cleaning step of the first row.

In some embodiments, in the cleaning step, if it is determined that the plurality of biochips are arranged in three or more rows, the cleaning step for the above-described plurality of biochips which are arranged in one row is performed sequentially on the three or more rows of biochips.

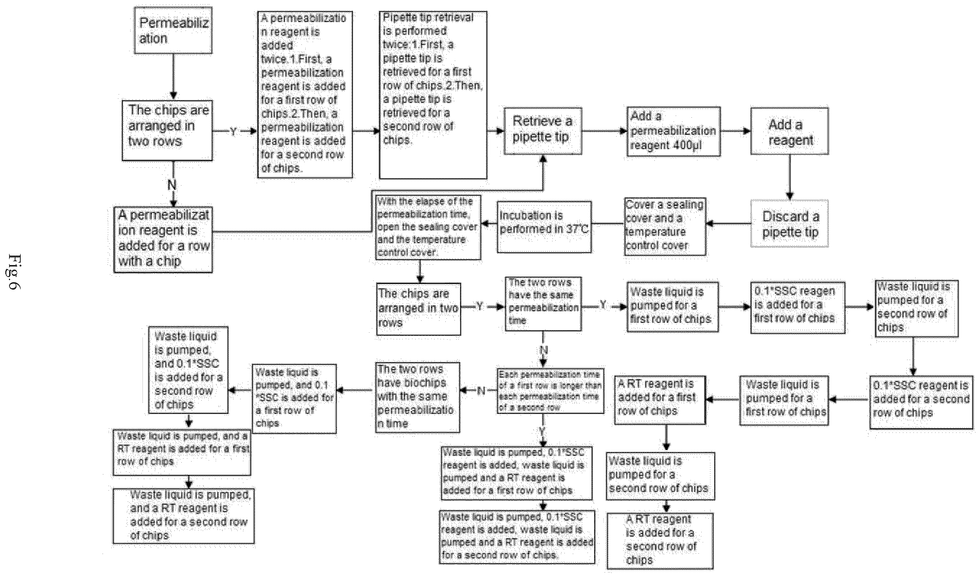

In some embodiments, as shown in Fig. 6, the tissue permeabilization step includes: determining whether the biochips are arranged in one row or two rows in the biochip kit:
step S1: if the biochips are arranged in only one row, add a permeabilization reagent to this row of biochips at the same time. After the permeabilization reagent is added, the pipette tip is discarded, and the biochip kit is covered with a sealing cover and a temperature control cover, and incubation is performed at a certain temperature (for example, 37°C). After completion of the permeabilization time, the sealing cover and the temperature control cover are opened. If this row of biochips has the same permeabilization time, simultaneously pump waste liquid of the permeabilization reagent, simultaneously add a certain amount of cleaning reagent with a low concentration (for example, 0.1*SSC), simultaneously pump waste liquid of the cleaning reagent and simultaneously add a reverse transcription reagent for this row of biochips.

step S2: if the biochips are arranged in two rows, and the two rows of biochips have the same permeabilization time, a permeabilization reagent (for example, 400µL) is first added to a first row of biochips for the first time. The permeabilization reagent (for example, 400µL) is added to a second row of biochips for the second time. After the permeabilization reagent is added, the pipette tip is discarded, and the biochip kit is covered with a sealing cover and a temperature control cover. Incubation is performed at a certain temperature (for example, 37°C), and the sealing cover and the temperature control cover are opened after completion of the permeabilization time. Waste liquid of the permeabilization reagent is pumped and a certain amount of cleaning reagent with a low concentration (for example, 0.1*SSC) is added for a first row, before waste liquid of the cleaning reagent is pumped and a certain amount of cleaning reagent with a low concentration (for example, 0.1*SSC) is added for a second row. Then, waste liquid of the cleaning reagent is pumped and the reverse transcription reagent is added for a first row. Next, waste liquid of the cleaning reagent is pumped and the reverse transcription reagent is added for a second row.

In some embodiments, in the tissue permeabilization step, if it is determined that the biochips are arranged in three or more rows and all the biochips have the same permeabilization time, the three or more rows of biochips are divided into multiple groups of biochips in two adjacent rows, or divided into at least one group of biochips in two adjacent rows and one row of biochips. Step S1 is performed on one row of biochips, and step S2 is performed sequentially on each of a plurality of groups of biochips in two adjacent rows or at least one group of biochips in two adjacent rows. That is, for each group of biochips in two adjacent rows, the permeabilization reagent is first added to a previous row of biochips, and then the permeabilization reagent is added to a next row of biochips. When the permeabilization time of the previous row of biochips is over, the permeabilization waste liquid reacted by the previous row of biochips is pumped and the cleaning reagent is added at the same time. When the permeabilization time of the next row of biochips is over, the permeabilization waste liquid reacted by the next row of biochips is pumped and the cleaning reagent is added at the same time. Next, the cleaning waste liquid of the previous row of biochips is pumped and the reverse transcription reagent is added at the same time. Then, the cleaning waste liquid of the next row of biochips is pumped and the reverse transcription reagent is added at the same time.

In some embodiments, as shown in Fig. 6, the tissue permeabilization step includes: determining whether the biochips are arranged in one row or two rows:
Step S3: if the biochips are arranged in only one row in the biochip kit, the permeabilization reagent is added to this row of biochips at the same time. After the permeabilization reagent is added, the pipette tip is discarded, and the biochip kit is covered with a sealing cover and a temperature control cover. Incubation is performed at a certain temperature (for example, 37°C), and the sealing cover and the temperature control cover are opened after completion of the permeabilization time. If this row of biochips do not have the same permeabilization time, the permeabilization time difference between every two adjacent biochips with different permeabilization time is not less than twice the time of a single pumping and injection process of the pipette mechanism. According to a temporal sequence of completion of the permeabilization time, pump waste liquid of the permeabilization reagent, add a certain amount of cleaning reagent with a low concentration (for example, 0.1*SSC), pump waste liquid of the cleaning reagent, add a reverse transcription reagent sequentially for each biochip among this row of biochips.

Step S4: when the biochips are arranged in two rows in the biochip kit, this row of biochips do not have the same permeabilization time, and the permeabilization time difference between every two adjacent biochips with different permeabilization time is not less than twice the time of a single pumping and injection process of the pipette mechanism. Each permeabilization time of the first row of biochips is not the same as each permeabilization time of the second row of biochips. The permeabilization reagent is added for the first row of biochips before the permeabilization reagent is added for the second row of biochips, and the time difference of adding the permeabilization reagent between the first row of biochips and the second row of biochips is the time of a single pumping and injection process. After the permeabilization reagent is added, the pipette tip is discarded, and the biochip kit is covered with a sealing cover and a temperature control cover. Incubation is performed at a certain temperature (for example, 37°C), and the sealing cover and the temperature control cover are opened with the elapse of the permeabilization time. According to a temporal sequence of completion of the permeabilization time, pump waste liquid of the permeabilization reagent, add a certain amount of cleaning reagent with a low concentration (for example, 0.1*SSC), pump waste liquid of the cleaning reagent and add a reverse transcription reagent sequentially for each biochip among the first row of biochips according to a temporal sequence of completion of the permeabilization time, before pumping waste liquid of the permeabilization reagent, adding a certain amount of cleaning reagent with a low concentration (for example, 0.1*SSC), pumping waste liquid of the cleaning reagent and adding a reverse transcription reagent sequentially for each biochip among the second row of biochips according to a temporal sequence of completion of the permeabilization time.

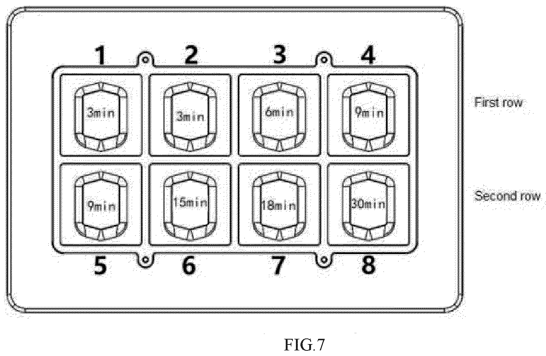

In some embodiments, as shown in Fig. 7, a plurality of biochips is arranged in two rows, and the two rows of biochips do not have the same permeabilization time. Moreover, the permeabilization time difference between every two adjacent biochips with different permeabilization time is not less than twice the time of a single pumping and injection process of the pipette mechanism, which is 1.5 minutes. The first row includes first to fourth biochips, and the second row includes fifth to eighth biochips. The first row and second row of biochips include biochips having the same permeabilization time, and the fourth biochip in the first row and the fifth biochip in the second row both have the permeabilization time of 9min. Step S5: first, the permeabilization reagent is added to the first row of biochips, and then, the permeabilization reagent is added to the second row of biochips. After the permeabilization reagent is added, the pipette tip is discarded, and the biochip kit is covered with a sealing cover and a temperature control cover. Incubation is performed at a certain temperature (for example, 37°C), and the sealing cover and the temperature control cover are opened after completion of the permeabilization time. In addition to the fourth and fifth biochips, according to a temporal sequence of completion of the permeabilization time, pump waste liquid of the permeabilization reagent, add a certain amount of cleaning reagent with a low concentration (for example, 0.1*SSC), pump waste liquid of the cleaning reagent and add a reverse transcription reagent sequentially for each biochip among the first row of biochips. Since the first and second biochips have the permeabilization time of 3min, the above-described steps are performed synchronously for the first and second biochips. Similarly, if some or all the biochips in one row have the same permeabilization time, some or all the biochips are operated synchronously. The permeabilization reagent is pumped and the cleaning reagent with a low concentration (for example, 0.1*SSC) is added for the fourth biochip in the first row, before the permeabilization reagent is pumped and the cleaning reagent with a low concentration (for example, 0.1*SSC) is added for the fifth biochip in the second row. Then, waste liquid of the cleaning reagent is pumped and the reverse transcription reagent is added for the fourth biochip in the first row. Next, waste liquid of the cleaning reagent is pumped and the reverse transcription reagent is added for the fifth biochip in the second row.

In some embodiments, the biochips are arranged in three or more rows, each biochip does not have the same permeabilization time, and the permeabilization time difference between every two adjacent biochips with different permeabilization time is not less than twice the time of a single pumping and injection process of the pipette mechanism. The three or more rows of biochips are divided into multiple groups of biochips in two adjacent rows, or divided into at least one group of biochips in two adjacent rows and one row of biochips. Step S3 is performed on one row of biochips, and step S4 or S5 is performed sequentially on each of a plurality of groups of biochips in two adjacent rows or at least one group of biochips in two adjacent rows.

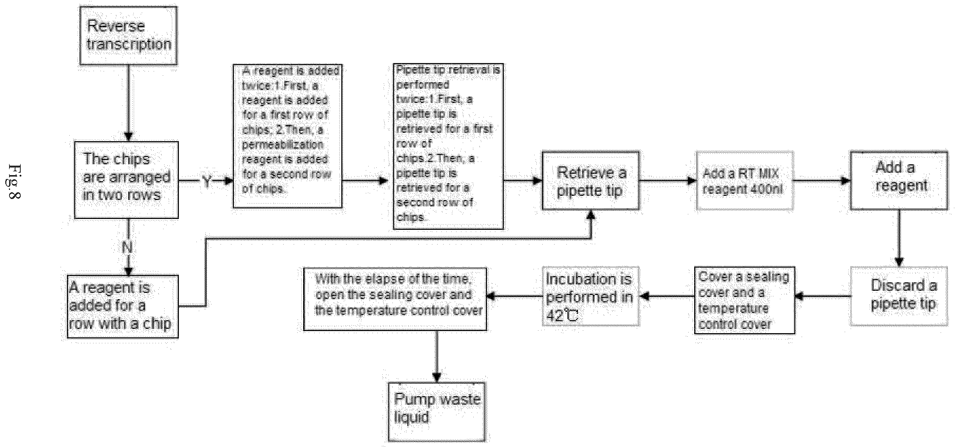

In some embodiments, as shown in Fig. 8, the reverse transcription step includes: determining whether the biochips are arranged in one row or two rows:

If the biochips are arranged in only one row, a reverse transcription mixed reagent is added for this row of biochips. After the reagent is added, the pipette tip is discarded, and a sealing cover and a temperature control cover are covered. Incubation is performed at a certain temperature (for example, 42°C), and the sealing cover and the temperature control cover are opened and waste liquid of the reverse transcription mixed reagent is pumped after time is up.

If the biochips are arranged in two rows, a reagent is added to the biochips twice. First, a reverse transcription mixed reagent is added to a first row of biochips. Then, a reverse transcription mixed reagent is added to a second row of biochips. After the reagent is added, the pipette tip is discarded, and a sealing cover and a temperature control cover are covered. Incubation is performed at a certain temperature (for example, 42°C), and the sealing cover and the temperature control cover are opened and waste liquid of the reverse transcription mixed reagent is pumped after incubation time is up.

In some embodiments, in the reverse transcription step, if the biochips are arranged in three or more rows, a reverse transcription mixed reagent is added sequentially to the three or more rows of biochips. After the reverse transcription mixed reagent is added, the pipette tip is discarded, and a sealing cover and a temperature control cover are covered. Incubation is performed at a certain temperature (for example, 42°C), and the sealing cover and the temperature control cover are opened and waste liquid of the reverse transcription mixed reagent is pumped after time is up.

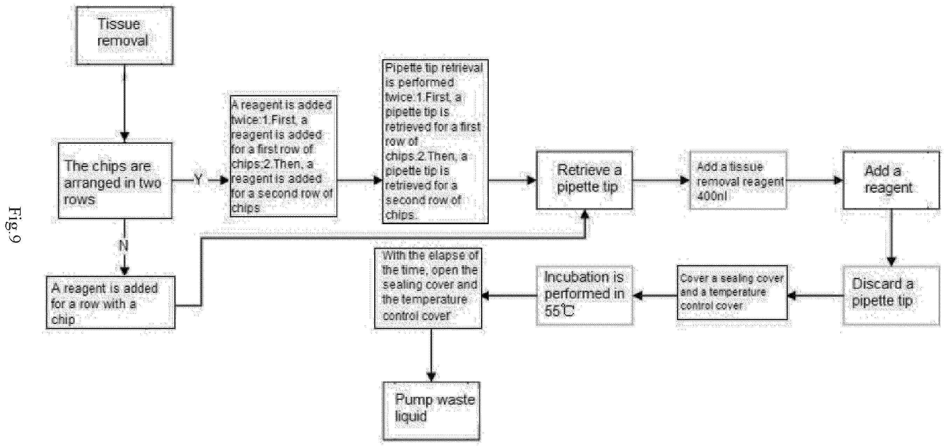

In some embodiments, as shown in Fig. 9, the tissue removal step includes: determining whether a plurality of biochips is arranged in one row or two rows:

If the biochips are arranged in only one row, add a tissue removal reagent to this row of biochips. After the tissue removal reagent is added, the pipette tip is discarded, and the biochip kit is covered with a sealing cover and a temperature control cover, and incubation is performed at a certain temperature (for example, 55°C). After completion of the incubation time, the sealing cover and the temperature control cover are opened, and the tissue removal waste liquid is pumped.

If the biochips are arranged in two rows, the tissue removal reagent is added to the first row, before the tissue removal reagent is added to the second row. After the tissue removal reagent is added, the pipette tip is discarded, and the biochip kit is covered with a sealing cover and a temperature control cover, and incubation is performed at a certain temperature (for example, 55°C). After completion of the incubation time, the sealing cover and the temperature control cover are opened, and waste liquid of the tissue removal reagent is pumped.

In some embodiments, in the tissue removal step, if the biochips are arranged in only three or more rows, the above-described tissue removal step for only one row of biochips is sequentially performed on only three or more rows of biochips.

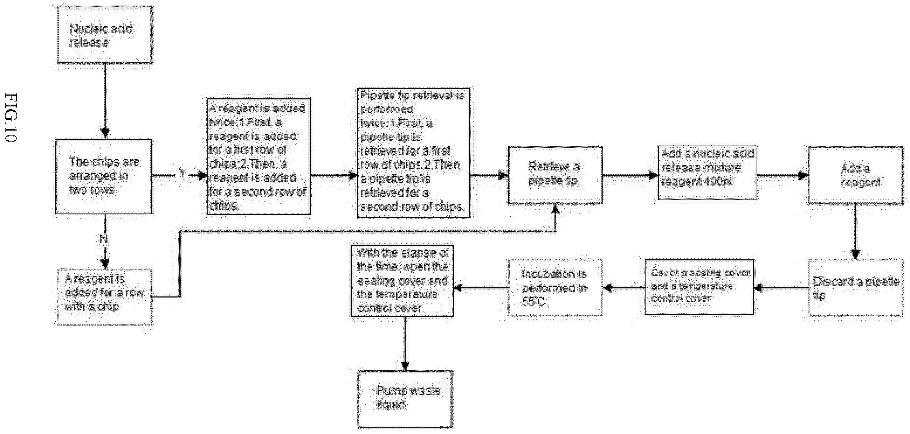

In some embodiments, as shown in Fig. 10, the nucleic acid release step includes: determining whether the biochips are arranged in one row or two rows:
If the biochips are arranged in one row, a nucleic acid release mixed reagent is added for this row of biochips. After the nucleic acid release mixed reagent is added, the pipette tip is discarded, and the biochip kit is covered with a sealing cover and a temperature control cover, and incubation is performed at a certain temperature (for example, 55°C). After completion of the incubation time, the sealing cover and the temperature control cover are opened, and waste liquid of the nucleic acid release mixed reagent is pumped.

If the biochips are arranged in two rows, the nucleic acid release mixed reagent is added to the first row, before the nucleic acid release mixed reagent is added to the second row. After the nucleic acid release mixed reagent is added, the pipette tip is discarded, and the biochip kit is covered with a sealing cover and a temperature control cover, and for example, incubation is performed at a certain temperature (for example, 55°C). After completion of the incubation time, the sealing cover and the temperature control cover are opened, and waste liquid of the nucleic acid release mixed reagent is pumped.

In some embodiments, in the nucleic acid release step, if the biochips are arranged in only three or more rows, the above-described nucleic acid release step for only one row of biochips is sequentially performed on three or more rows of biochips.

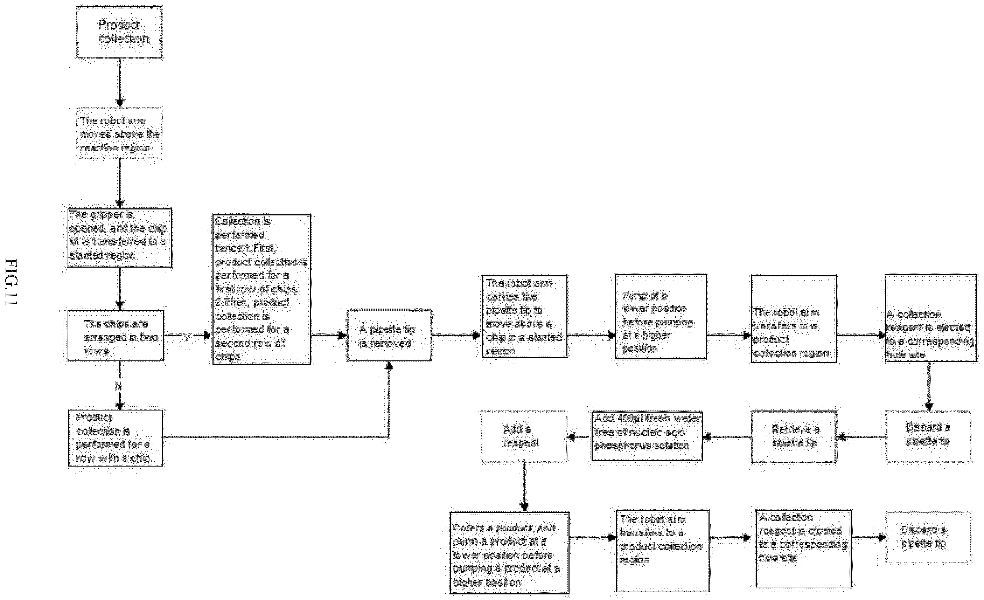

In some embodiments, as shown in Fig. 11, the product collection step includes: wherein the system controls the gripper mechanism to move above the reaction region, open the gripper and transfer the biochip kit to the slanted zone, and determines whether the biochips in the biochip kit are one row or two rows:
If the biochips are arranged in only one row, all the biochip products are collected once. The collection process is as follows: first, a pipette tip is retrieved, and the pipette mechanism carries the pipette tip to move above the biochip on the slanted region, wherein the biochip kit is inclined. A product located at a lower position in the biochip kit is retrieved before a product located at a higher position in the biochip kit is retrieved. Then, the pipette mechanism reaches the product collection region, the collection reagent is released to the corresponding hole site, and the empty pipette tip is discarded into a dust bin. Then, a pipette tip is retrieved, a certain amount of nuclease-free water (for example, 400µL) is added for cleaning, and a cleaning product is collected. A product at a lower position in the biochip kit is pumped before a product at a higher position in the biochip kit is pumped. Then, the pipette mechanism reaches the product collection region, the collection reagent is released to a corresponding hole site, and then the empty pipette tip is discarded into a dust bin.

If the biochips are arranged in two rows, the above-described product collection step of the biochips which are arranged in only one row is performed sequentially on the first row of biochips and the second row of biochips.

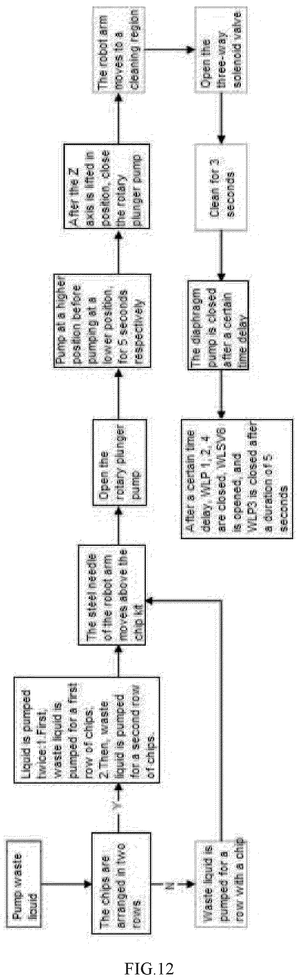

In some embodiments, as shown in Fig. 12, the waste liquid pumping step includes: determining that a plurality of biochips are arranged in one row or two rows:
If the plurality of biochips are arranged in one row, the waste liquid pumping mechanism is moved above the biochip kit, a pump of the waste liquid pumping mechanism is started to first pump the waste liquid from a higher position in the biochip kit for a period of time (for example, 5 seconds) and then pump the waste liquid from a lower position in the biochip kit for a period of time (for example, 5 seconds) through a waste liquid pumping member, raising the waste liquid pumping member along a Z axis and closing the pump, and moving the waste liquid pumping mechanism to a cleaning region, and the three-way valve is turned on to clean the waste liquid pumping member for a period of time (for example, 3 seconds).

If the plurality of biochips is arranged in two rows, the waste liquid reacted by the first row of biochips is pumped before the waste liquid reacted by the second row of biochips is pumped, wherein the pumping step is the same as the above-described pumping step when the plurality of biochips are arranged in one row.

In some embodiments, in the product collection step, if the biochips are arranged in three or more rows, the above-described product collection step for the biochips which are arranged in only one row is sequentially performed on three or more rows of biochips.

In some embodiments, the waste liquid pumping mechanism may include a pump and a needle, and may also include a pump and a disposable pipette tip. The pumping member of the waste liquid pumping mechanism may be a needle or a disposable pipette tip, and the pump may be an air pump, a rotary plunger pump, a diaphragm pump or a plunger pump.

In some embodiments, the performing stage includes a test operation to detect the reaction conditions of a method for automatically controlling a biochip and an automatic control system implementing the method, so as to better perform the capture and product collection of spatial omics.

In some embodiments, the test operation uses steps similar to spatial omics, and the main difference lies in that: when a test biochip with a low cost (for example, a fluorescent biochip) is used, product collection is not required, and unloading observation is directly performed after tissue removal and cleaning.

In some embodiments, in the test operation, the permeabilization conditions of the test biochips in the same biochip kit are set to be inconsistent. Specifically, the upper and lower rows of test biochips have different permeabilization time and some test biochips in the upper and lower rows have the same permeabilization time, as shown in Fig. 7, for example.

In some embodiments, it is determined whether it is necessary to adjust the reaction conditions of a method for automatically controlling a biochip and an automatic control system implementing the method according to the permeabilization effect observed during unloading.

In some embodiments, the size of the biochip on which a target tissue is carried may be customized. Thus, the adaptation flexibility of the biochip is improved.

In some embodiments, the method and the automatic control system according to the present disclosure are applied to multi-omics labeling, multi-omics capture and/or pathological labeling. In some embodiments, as shown in Fig. 1, the following steps may be adopted for the spatio-temporal biochip by the automatic control system to realize transcriptome capture and product collection.

Outside the system, the information of a single biochip is correlated with the information of the biochip kit by scanning a code, for example, the permeabilization time of each biochip is manually input, and a plurality of biochips are arranged in the biochip kit sequentially in an ascending order according to the permeabilization time, for example, a plurality of biochips at least arranged in at least one row. For example, a performing consumable (a disposable pipette tip, a reagent and the like) is manually placed. By selecting a performing process of the software interface and clicking a start button, the system enters an automatic running process.

Starting from scanning a code, scan a QR code outside the biochip kit, and scan a biochip QR code in the biochip kit to determine whether the biochip position/biochip number/biochip QR code is matched with the information in the system (manually entered/obtained from a docking system). If not matched, the user is reminded by a pop-up window that: if the biochip information is not matched with the information in the system, the process ends; if matched, enter a next process;
When scanning a code is completed and the biochip information is matched with the system, enter the step of determining a state of the biochip kit in the system. If some of the existing biochip kits in the system are not in the reverse transcription or nucleic acid release stage, the user is reminded that: it is still necessary to wait for a duration of X before transferring the biochip kit to a certain reaction region. If all the biochip kits in the system are in the reverse transcription or nucleic acid release step, enter a next flow.

It is determined whether there is a vacancy in the reaction region. If all the reaction regions are occupied, the biochip kit will enter a waiting region, and the user is reminded that: it is still necessary to wait for a duration of Y before transferring the biochip kit to a certain reaction region. If there is a vacancy in the reaction region, enter a next flow.

As shown in Fig. 1, the biochip kit is gripped from the loading region by the gripping mechanism, for example, a gripper, and the biochip kit is transferred to the reaction region. Then, the following above-described steps are performed sequentially: incubation step→cleaning step-tissue permeabilization step-reverse transcription step-cleaning step-tissue removal step-cleaning step→cDNA release step→cDNA collection step.

In some embodiments, the present disclosure provides a method for automatically controlling a biochip. The automatic control system of a biochip is used for labeling and capture of proteome as well as product collection, and the step of realizing labeling and capture of proteome as well as product collection includes the following operation steps:

Outside the system, the information of a single biochip is correlated with the information of the biochip kit by scanning a code, for example, the permeabilization time of each biochip is manually input, and a plurality of biochips are arranged in the biochip kit sequentially in an ascending order according to the permeabilization time, for example, a plurality of biochips are at least arranged in at least one row. For example, a performing consumable (a disposable pipette tip, a reagent and the like) is manually placed. By selecting a performing process of the software interface and clicking a start button, the system enters an automatic running process. The performing process of the software interface is selected, and the start button is clicked to enter an automatic running process:
Starting from scanning a code, scan a QR code outside the biochip kit, and scan a biochip QR code in the biochip kit to determine whether the biochip position/biochip number/biochip QR code is matched with the information in the system (manually entered/obtained from a docking system). If not matched, the user is reminded by a pop-up window that: if the biochip information is not matched with the information in the system, the process ends; if matched, enter a next process.

When scanning a code is completed and the biochip information is matched with the system, enter the step of determining a state of the biochip kit in the system. If some of the existing biochip kits in the system are not in the reverse transcription or nucleic acid release stage, the user is reminded that: it is still necessary to wait for a duration of X before transferring the biochip kit to a certain reaction region. If all the biochip kits in the system are in the reverse transcription or nucleic acid release step, enter a next flow.

It is determined whether there is a vacancy in the reaction region. If all the reaction regions are occupied, the biochip kit will enter a waiting region, and the user is reminded that: it is still necessary to wait for a duration of Y before transferring the biochip kit to a certain reaction region. If there is a vacancy in the reaction region, enter a next flow.

As shown in Fig. 1, the biochip kit is gripped from the loading region by the gripping mechanism, for example, a gripper, and the biochip kit is transferred to the reaction region. Then, the following steps are performed sequentially:
Cleaning: retrieve a pipette tip, add a certain amount of cleaning reagent such as PBST and WB, clean the biochip, discard the pipette tip, and then pump waste liquid of the cleaning reagent.

Blocking: the blocking liquid is sucked by the pipette mechanism, then the pipette mechanism carries the pipette tip that has sucked the blocking liquid to add the blocking liquid to the biochip, and after blocking is completed, waste liquid of the blocking liquid is pumped by the waste liquid pumping mechanism.

Antibody incubation: the antibody reagent is sucked by the pipette mechanism, and then the antibody reagent is added to the biochip by the pipette tip that has sucked the antibody reagent carried by the pipette mechanism, and the antibody reagent waste liquid is pumped by the waste liquid pumping mechanism after incubation is completed.

Cleaning: retrieve a pipette tip, add a certain amount of cleaning reagent such as PBST and WB, clean the biochip, discard the pipette tip, and then pump waste liquid of the cleaning reagent.

Drying: dry the biochip after the waste liquid is pumped in the biochip reaction region.

The labeling and capture of proteome as well as product collection also include steps such as tissue permeabilization, reverse transcription, tissue removal, nucleic acid release and product collection, which are the same as corresponding steps of transcriptome capture and product collection.

According to various embodiments of the present disclosure, the method and automatic control system of a biochip may bring at least one of the following technical advantages:
Manual intervention is not required in the whole performing process.

The software interface may display a currently performed step and state in real time and may display estimated remaining performing time. The operator may predict and arrange operations in advance as it depends, so as to save the manpower.

A plurality of full biochip samples may be processed at the same time, with a high daily flux.

It is possible to incorporate a high utilization rate and a minimized reagent consumption of a biochip.

100% biochip surface utilization is achieved.

A biochip with a customized size is supported, and biochemical reaction of a planar biochip and separation and collection of surface products are completed.

The present disclosure also provides an automatic control system for automatically performing the steps in the above embodiments.

Finally, it is to be noted that: the above embodiments are only for describing the technical solution of the present disclosure, rather than limiting the same. Although the present disclosure has been described in detail in conjunction with the foregoing embodiments, those skilled in the art should understand that: it is still possible to modify the technical solutions recited in the foregoing various embodiments, or make equivalent substitutions to some of the technical features. Moreover, these modifications or substitutions do not make the essence of the corresponding technical solutions depart from the spirit and scope of the technical solutions in various embodiments of the present disclosure.

## Claims

1. A method for automatically controlling a biochip, comprising operation, the operation comprising:
carrying a suction member by means of a pipette mechanism to suck a reagent; and
carrying the suction member that has sucked the reagent by means of the pipette mechanism to add the reagent to the biochip.

2. The method according to claim 1, wherein the operation comprising at least one of following steps:
incubation: the pipette mechanism carries the suction member to suck an incubation reagent, and the pipette mechanism carries the suction member that sucked the incubation reagent to add the incubation reagent to the biochip;
tissue permeabilization: the pipette mechanism carries the suction member to suck a permeabilization reagent, and the pipette mechanism carries the suction member that has sucked the permeabilization reagent to add the permeabilization reagent to the biochip;
reverse transcription: the pipette mechanism carries the suction member to suck a reverse transcription reagent, and the pipette mechanism carries the suction member that has sucked the reverse transcription reagent to add the reverse transcription reagent to the biochip; and
tissue removal: the pipette mechanism carries the suction member to suck a tissue removal reagent, and the pipette mechanism carries the suction member that has sucked the tissue removal reagent to add the tissue removal reagent to the biochip.

3. The method according to claim 2, wherein in each step of the operation, waste liquid is pumped by a waste liquid pumping mechanism.

4. The method according to claim 2, wherein after the tissue removal step, comprising at least one of following steps:
nucleic acid release: the pipette mechanism carries the suction member to suck a nucleic acid release reagent, and the pipette mechanism carries the suction member that has sucked the nucleic acid release reagent to add the nucleic acid release reagent to the biochip to complete the nucleic acid release; and
product collection: the pipette mechanism carries the suction member to suck the released nucleic acid and store the released nucleic acid.

5. The method according to claim 4, wherein a cleaning step is performed at least one of:
between the incubation step and the tissue permeabilization step;
between the tissue permeabilization step and the reverse transcription step;
between the reverse transcription step and the tissue removal step; and
between the tissue removal step and the nucleic acid release step.

6. The method according to claim 5, wherein the cleaning step comprises: the pipette mechanism carries the suction member to suck a cleaning reagent, and the pipette mechanism carries the suction member that has sucked the cleaning reagent to add the cleaning reagent to the biochip, and the waste liquid pumping mechanism pumps the cleaning waste liquid.

7. The method according to any one of claims 2 to 6, wherein each step of the operation is automatically performed.

8. The method according to any one of claims 2 to 7, wherein in each step of the operation, a current step and/or a current state are displayed to the user in real time.

9. The method according to any one of claims 1 to 8, wherein before the operation, a plurality of biochips is arranged in a biochip kit, so that the information of the plurality of biochips is associated with the information of the biochip kit by scanning, and the permeabilization time of the plurality of biochips is input into the system.

10. The method according to any one of claims 1 to 9, wherein the biochip on which a target tissue is carried is provided with a customized size.

11. The method according to any one of claims 2 to 10, wherein the plurality of biochips is sequentially arranged in at least one row in the biochip kit.

12. The method according to claim 11, wherein if the plurality of biochips has the same permeabilization time:
in the case that a plurality of biochips is arranged in one row, simultaneously pumping waste liquid of the permeabilization reagent, simultaneously adding the cleaning reagent, simultaneously pumping waste liquid of the cleaning agent, and simultaneously adding the reverse transcription reagent sequentially for the one row of biochips; and
in the case that the plurality of biochips is arranged in two rows, pumping waste liquid of the permeabilization reagent and adding the cleaning reagent for the first row of biochips before pumping waste liquid of the permeabilization reagent and adding the cleaning reagent for the second row of biochips; then pumping waste liquid of the cleaning reagent and adding the reverse transcription reagent for the first row of biochips before pumping waste liquid of the cleaning reagent and adding the reverse transcription reagent for the second row of biochips.

13. The method according to claim 11, wherein if the plurality of biochips does not have the same permeabilization time and the plurality of biochips are arranged sequentially in an ascending order according to the permeabilization time,
in the case that the plurality of biochips are arranged in one row, pumping waste liquid of the permeabilization reagent, adding the cleaning reagent, pumping waste liquid of the cleaning reagent, adding the reverse transcription reagent sequentially for the plurality of biochips according to a temporal sequence of completion of the permeabilization time;
in the case that the plurality of biochips are arranged in two rows, and the two rows of biochips do not have the same permeabilization time therebetween, pumping waste liquid of the permeabilization reagent, adding the cleaning reagent, pumping waste liquid of the cleaning agent, and adding the reverse transcription reagent sequentially for biochips in the first row of biochips according to a temporal sequence of completion of the permeabilization time before pumping waste liquid of the permeabilization reagent, adding the cleaning reagent, pumping waste liquid of the cleaning agent, and adding the reverse transcription reagent sequentially for biochips in the second row of biochips according to a temporal sequence of completion of the permeabilization time; and
in the case that the plurality of biochips are arranged in two rows, if the two rows of biochips have the same permeabilization time therebetween, first pumping waste liquid of the permeabilization reagent, adding the cleaning reagent, pumping waste liquid of the cleaning agent and adding the reverse transcription reagent sequentially for other biochips than the biochips having the same time among the first row of biochips according to a temporal sequence of completion of the permeabilization time; after pumping waste liquid of the permeabilization reagent and adding the cleaning reagent for the biochips having the same time among the first row of biochips, pumping waste liquid of the permeabilization reagent and adding the cleaning reagent for the biochips having the same time among the second row of biochips; after pumping waste liquid of the cleaning agent and adding the reverse transcription reagent for the biochips having the same time among the first row of biochips, pumping waste liquid of the cleaning agent and adding the reverse transcription reagent for the biochips having the same time among the second row of biochips; then pumping waste liquid of the permeabilization reagent, adding the cleaning reagent, pumping waste liquid of the cleaning agent and adding the reverse transcription reagent sequentially for other biochips among the second row of biochips according to a temporal sequence of completion of the permeabilization time.

14. The method according to claim 12 or 13, comprising a waste liquid pumping step, the waste liquid pumping step comprising:
in the case that the plurality of biochips are arranged in one row, moving the waste liquid pumping mechanism above the biochip kit, starting a pump of the waste liquid pumping mechanism to first pump the waste liquid at a higher position in the biochip kit and then pump the waste liquid at a lower position in the biochip kit through a waste liquid pumping member, raising the waste liquid pumping member along a Z axis and closing the pump, and moving the waste liquid pumping mechanism to a cleaning region for cleaning; and
in the case that the plurality of biochips are arranged in two rows, pumping the waste liquid reacted by the first row of biochips before pumping the waste liquid reacted by the second row of biochips, wherein the pumping step is the same as the pumping step when the plurality of biochips are arranged in one row.

15. The method according to any one of claims 9 to 14, wherein after inputting the permeabilization time of each biochip, the system scans the information of the biochip kit and the information of the biochip in the biochip kit to determine whether the position of the biochip in the biochip kit, the number of the biochip and the information of the biochip are matched with the information in the system;
if not matched, remind the user;
if matched, determine the states of the existing biochip kits in the system:
if some of the existing biochip kit are not in the reverse transcription step or the nucleic acid release step, remind the user that it is still necessary to wait for a duration of X before transferring the biochip kit to the reaction region to perform the operation; and
if all the existing biochip kits are in the reverse transcription step or the nucleic acid release step, determine whether there is a vacancy in the reaction region; if there is no vacancy, remind the user that it is still necessary to wait for a duration of Y before transferring the biochip kit to the reaction region; and if there is a vacancy, transfer the biochip kit to the reaction region to perform the operation.

16. The method according to any one of claims 10 to 15, wherein between the step of placing the biochip in the biochip kit and the operation, the biochip kit is transferred to the reaction region by a gripper mechanism to perform the operation, and the gripper mechanism and the pipette mechanism work independently of each other.

17. The method according to claim 16, wherein in each step of the operation, neither the gripper mechanism nor the pipette mechanism contacts the biochip.

18. The method according to claim 16 or 17, wherein covering a sealing cover and/or a temperature control cover and opening a sealing cover and/or a temperature control cover are performed on the biochip kit by the gripper mechanism.

19. The method according to claim 4, wherein in the product collection step, the biochip kit is first transferred to a slanted region by the gripper mechanism, and product collection is conducted in the slanted region.

20. The method according to claim 4 or 19, wherein in the product collection step, the pipette mechanism carries a suction member to suck a reagent, the pipette mechanism carries the suction member that has sucked the reagent to flush the biochip in the biochip kit, and the pipette mechanism carries the suction member to perform product collection.

21. The method according to any one of claims 1 to 20, comprising a step of retrieving a suction member by the pipette mechanism before the operation, the step comprising: determining whether a current column in a suction member container satisfies the conditions for retrieving a suction member, in the case that the number and position satisfy the conditions, the pipette mechanism moves above the row of suction members and drops along the height direction, and at the same time the row of suction members are retrieved; if the current column does not satisfy the conditions for retrieving a suction member, a next column is polled until polling of the suction member container is completed, and polling of the next suction member container begins until polling of all the suction member containers is completed; and if there is still not a column that satisfies the conditions for retrieving a suction member, the user is reminded to replenish a suction member.

22. The method according to any one of claims 1 to 21, wherein the step of sucking a reagent by the pipette mechanism comprises:
the pipette mechanism carries the suction member to transfer to above the reagent region and the height of the suction member decrease, a reagent level is detected:
if a reagent is not detected, remind the user to replenish a reagent; and
if a reagent is detected, determine whether a surplus amount satisfies an amount to be sucked by the carried suction member:
if not, remind the user to replenish a reagent; and
if yes, determine whether the reagent to be added is a cleaning reagent:
if it is a cleaning reagent, the heights of all the suction members decrease to suck the cleaning reagent together; and
if it is not a cleaning reagent, the heights of all the suction members decrease respectively to suck the reaction reagent.

23. The method according to any one of claims 1 to 22, wherein the method is applied to at least of: labeling or capturing of transcriptomics, proteomics, metabonomics, lipidomics as well as pathological labeling.

24. A system for automatically controlling a biochip, comprising a biochip kit, a reaction region, a pipette mechanism and a gripper mechanism, configured to perform the method according to any one of claims 1 to 23.
